# EUROPÄISCHE PATENTSCHRIFT

(11) **EP 1 047 655 B1**
(45) Veröffentlichungstag und Bekanntmachung des Hinweises auf die Patenterteilung: **07.05.2003**
(21) Anmeldenummer: 99904776.4
(22) Anmeldetag: 14.01.1999
(51) Int. Cl.: C07C 45/50, C07C 29/16, B01J 31/20, B01J 31/18

(54) **VERFAHREN ZUR HERSTELLUNG VON ALDEHYDEN**
METHOD FOR THE PRODUCTION OF ALDEHYDES
PROCEDE DE PRODUCTION D'ALDEHYDES

(30) Priorität: 16.01.1998 DE 19801437
(43) Veröffentlichungstag der Anmeldung: 02.11.2000
(73) Patentinhaber: BASF AKTIENGESELLSCHAFT, 67056 Ludwigshafen (DE)
(72) Erfinder: GEISSLER, Bernhard, D-67281 Kirchheim (DE); RÖPER, Michael, D-67157 Wachenheim (DE); ZELLER, Edgar, D-68163 Mannheim (DE); PACIELLO, Rocco, D-67098 Bad Dürkheim (DE); DECKER, Jürgen, D-54292 Trier (DE); VOSS, Hartwig, D-67227 Frankenthal (DE); MAHR, Norbert, D-67117 Limburgerhof (DE)
(86) Internationale Anmeldenummer: EP9900187
(87) Internationale Veröffentlichungsnummer: WO99036382

(56) Entgegenhaltungen:
- DE-A- 3 727 704
- DE-A- 19 603 201
- US-A- 3 594 425
- US-A- 4 235 744

## Beschreibung

Die Erfindung betrifft ein Verfahren zur Herstellung von Aldehyden und Alkoholen durch Hydroformylierung von Olefinen in Gegenwart von Komplexkatalysatoren enthaltend ein Metall der Gruppe VIII des Periodensystems der Elemente und als Ligand ein phosphorfreies, derivatisiertes, im wesentlichen nicht wasserlösliches hochmolekulares Polyamin und Rückführung des im Sumpf der Destillation des Reaktionsgemisches verbleibenden Katalysatorkomplexes in die Hydroformylierungsreaktion.

Die Hydroformylierung von Olefinen mit Kohlenmonoxid und Wasserstoff in Gegenwart von Übergangsmetall-Katalysatoren ist bereits sehr gut untersucht. Während α-Olefine sehr gut mit Rhodium-haltigen Phosphin-modifizierten Katalysatoren hydroformylierbar sind (vgl. J. Falbe, Ed.: New Syntheses With Carbon Monoxide, Springer, Berlin 1980, S. 55 ff), ist dieses Katalysatorsystem für interne und interne, verzweigte Olefine sowie für Olefine mit mehr als 7 Kohlenstoffatomen wenig geeignet (vgl. Falbe, S. 95ff). So werden interne Kohlenstoff-Kohlenstoff-Doppelbindungen nur sehr langsam in Gegenwart eines derartigen Katalysators hydroformyliert. Da die Abtrennung des Hydroformylierungsproduktes vom homogen im Reaktionssystem gelösten Katalysator in der Regel destillativ erfolgt und sich der Siedepunkt des bei der Hydroformylierung gebildeten Aldehyds mit zunehmender Kohlenstoffzahl und Kettenlänge auf Temperaturen erhöht, bei denen sich der Rhodium-Phosphin-haltige Katalysator zersetzt, ist diese Hydroformylierungsmethode für die Hydroformylierung von Olefinen mit mehr als 7 Kohlenstoffatomen nicht wirtschaftlich.

Hingegen lassen sich interne und interne, verzweigte Olefine vorteilhaft mit sogenanntem "nacktem" Rhodium hydroformylieren, d.h. mit homogen im Hydroformylierungsmedium gelösten Rhodiumverbindungen, die nicht mit Phosphor-haltigen Liganden, wie Phosphinen oder Phosphiten, modifiziert sind. Solche nicht mit Phosphinen oder Phosphiten modifizierte Rhodium-Katalysatoren und deren Eignung als Katalysator zur Hydroformylierung der zuvor genannten Klassen von Olefinen sind bekannt (siehe Falbe e.c., S. 38ff). Die Begriffe "nacktes Rhodium" oder "nackte" Rhodium-Katalysatoren werden in dieser Anmeldung für Rhodium-Hydroformylierungskatalysatoren gebraucht, die im Gegensatz zu herkömmlichen Rhodium-Hydroformylierungskatalysatoren unter den Bedingungen der Hydroformylierung nicht mit Phosphor-haltigen Liganden, wie Phosphin- oder Phosphit-Liganden modifiziert sind. Als Liganden in diesem Sinn werden nicht Carbonyl- oder Hydrido-Liganden verstanden. Es wird in der Fachliteratur (s. Falbe 1.c., S. 38ff) angenommen, daß die Rhodiumverbindung HRh(CO)₄ die katalytisch aktive Rhodiumspezies bei der Hydroformylierung mit "nackten Rhodium-Katalysatoren" ist, obgleich dies aufgrund der vielen in der Hydroformylierungszone nebeneinander ablaufenden Chemismen nicht eindeutig bewiesen ist. Lediglich der Einfachheit halber wird auch hier von dieser Annahme Gebrauch gemacht. Die "nackten" Rhodium-Katalysatoren bilden sich unter den Bedingungen der Hydroformylierungsreaktion aus Rhodium-Verbindungen, z.B. Rhodiumsalzen, wie Rhodium(III)chlorid, Rhodium(III)nitrat, Rhodium(II)acetat, Rhodium(III)acetylacetonat, Rhodium(III)sulfat oder Rhodium-(III)ammoniumchlorid, aus Rhodiumchalkogeniden, wie Rhodium(III)oxid oder Rhodium(III)sulfid, aus Salzen von Rhodiumsauerstoffsäuren, beispielsweise den Rhodaten, aus Rhodium-Carbonylverbindungen, wie Rhodiumdicarbonylacetylacetonat, Cyclooctadien-Rhodium-acetat oder -chlorid in Gegenwart von CO/H₂-Gemischen, die gemeinhin als Synthesegas bezeichnet werden. Zur Durchführung von Hydroformylierungen mit "nacktem" Rhodium sei an dieser Stelle beispielhaft auf die folgenden Literaturstellen verwiesen: US-A 4 400 547; DE-A 33 38 340; DE-A 26 04 545; WO 82/03856; Chem. Ber. 102, 2238 (1969); Tetrahedron Lett. 29, 3261 (1968); Hydrocarbon Process. 85 - 86 (1975) EP-A 588 225, wo 95/25080, EP-A 695 734, WO 96/16012, WO 97/30016, DE-A 19608559, EP-A 885 183.

Allerdings hat auch die Hydroformylierung mit "nacktem" Rhodium den Nachteil, daß sich der thermolabile Rhodium-Katalysator (vgl. US 4 400 547) infolge der thermischen Belastung bei der destillativen Aufarbeitung des Hydroformylierungsproduktes teilweise zu metallischem Rhodium zersetzt, das sich an den Wandungen des Reaktors und Rohrleitungen abscheidet. Das ausgefallene Rhodiummetall kann nicht wieder in die Hydroformylierungsreaktion zurückgeführt werden, da es unter den Hydroformylierungsbedingungen nicht in die katalytisch aktive Rhodiumverbindung umgewandelt werden kann. Die aus diesem chemischen Verhalten der "nackten" Rhodium-Katalysatoren resultierenden Rhodiumverluste haben bislang eine größere industrielle Anwendung dieses Verfahrens verhindert.

In DE-A 33 38 340 und US 4 400 547 werden Verfahren zur Hydroformylierung mittels "nackter Rhodium-Katalysatoren" beschrieben, bei denen zur Verhinderung der Rhodiumabscheidungen dem Reaktionsaustrag der Hydroformylierung ein Phosphin oder Phosphit zugesetzt wird, welche den Rhodium-Katalysator durch Bildung von Phosphin- oder Phosphit-Komplexen vor einer thermischen Zersetzung im Zuge der destillativen Aufarbeitung des Hydroformylierungsaustrags schützt. Nach Abschluß der Destillation wird der rhodiumhaltige Destillationssumpf mit einem Oxidationsmittel behandelt, wobei das Rhodium in katalytisch aktiver Form aus den betreffenden Phosphin- oder Phosphit-Komplexen freigesetzt wird und die Phosphin- oder Phosphitliganden zu den entsprechenden unter Hydroformylierungsbedingungen keine Rhodium-Komplexe bildenden Phosphinoxiden und Phosphaten oxidiert werden. Der oxidierte Destillationssumpf wird dann erneut als Katalysator zur Hydroformylierung eingesetzt. Die bei der Oxidation entstandenen oxidierten Phosphorverbindungen stören bei der Hydroformylierung in der Regel nicht, jedoch reichern sich verfahrensbedingt die oxidierten Phosphorverbindungen in diesem Hydroformylierungskreislauf an. Neben diesen oxidierten Phosphorverbindungen reichern sich verfahrensbedingt auch die entstehenden hochsiedenden Komponenten (z.B. Aldolkondensationsprodukte der Aldehyde) im Katalysatorkreislauf an, da diese nicht mit den Produkten abdestilliert werden können und somit eine Ausschleusung aus dem Katalysatorkreislauf erfordern.

Gemäß US-A 4 252 678 werden homogene, kolloidale Metallcluster der Elemente Rhodium, Ruthenium, Osmium und Iridium, die an ein Polymer gebunden sind, als Katalysatoren für die Hydroformylierung verwendet. Als hierzu verwendbare Polymere werden Vinylpolymere genannt, die Alkenyl-, Phosphin-, Phosphinoxid-, Arsin-, Isonitril- und Isocyanat-Gruppen enthalten, insbesondere Copolymere des Styrols, des Ethylens und deren Derivaten mit Butadien, Isopren, Cyclopentadien, p-Styryldiphenylphosphin und p-Styroldiphenylphosphinoxid. Die Herstellung dieser Metallcluster durch die thermische Zersetzung von Carbonylclustern der betreffenden Metalle gestaltet sich sehr aufwendig, weshalb sich dieses Verfahren nicht durchsetzen konnte.

Liu et al (Macromol. Symp. 105, 179 (1996)) beschreiben die Hydroformylierung von Propylen mit an Polyvinylpyrrolidon gebundenen Rhodiumclustern, die kolloid in Wasser dispergiert sind. Da Polyvinylpyrrolidon im Hydroformylierungsmedium nicht löslich ist, muß bei Anwendung dieses Katalysators im Zwei-Phasen-System Wasser/organisches Hydroformylierungsmedium gearbeitet werden, wodurch wertvoller Hochdruckreaktionsraum im Reaktor durch Wasser belegt wird, mit der Folge einer unbefriedigenden Raum-Zeit-Ausbeute bezogen auf das gesamte Reaktorvolumen. Darüberhinaus sind diese Katalysatoren sehr luftempfindlich und werden beim Kontakt mit Luft im Zuge der Katalysatorrückführung irreversibel desaktiviert.

DE-A 19603201 betrifft ein Verfahren zur Herstellung von Aldehyden oder Aldehyden und Alkoholen durch Hydroformylierung von Olefinen mit mehr als 3 Kohlenstoffatomen umfassend eine Hydroformylierungsstufe, in der man mittels eines in einem homogen Reaktionsmedium gelösten Rhodiumkatalysators das Olefin bei 50 bis 1000 bar und einer Temperatur von 50 bis 180°C hydroformyliert und eine Katalysatorrückgewinnungsstufe durch Extraktion des Rhodiumkatalysators, indem man
(a) die Hydroformylierung in Gegenwart eines Rhodiumkomplexes durchführt, der als Ligand eine phosphorfreie, mehrzähnige, zur Komplexbildung mit Metallen der Gruppe VIII des periodischen Systems befähigte, organische Stickstoffverbindung aufweist, die zusätzlich mindestens einen, durch eine schwache Säure protonierbaren tertiären Stickstoffrest enthält,
(b) den Austrag der Hydroformylierungsstufe, gegebenenfalls nach Abtrennung oder teilweiser Abtrennung der Aldehyde und Alkohole einer Extraktion mit einer wäßrigen Lösung einer destillierbaren Säure unterwirft,
(c) das wäßrige Säureextrakt in Gegenwart eines organischen Lösungsmittels oder Lösungsmittelgemisches, das unter den Hydroformylierungsbedingungen inert ist, unter Abdestillieren der wäßrigen Säure einer Hitzebehandlung, durch die der Komplex entprotoniert und in die organische Phase überführt wird, unterwirft und
(d) die organische, den Katalysatorkomplex enthaltende Phase in die Hydroformylierungsstufe zurückführt.

US-A 4,235,744 betrifft Carbonylierungsverfahren, einschließlich der Hydroformylierung, mittels homogener Kobalt- oder Rutheniumkatalysatoren, die mit einem sogenannten Polyaminliganden komplexiert sind. Als sogenannte Polyaminliganden werden verschiedenerlei Diamine und Polyvinylpyrrolidon eingesetzt. Im einzigen Beispiel von US-A 4,235,744, das einen Polyvinylpyrrolidon-Liganden verwendet (Beispiel 19, Table I), wird Methanol als Lösungsmittel und gleichzeitig als Reagenz eingesetzt. Dementsprechend findet bei der Umsetzung von Acrylnitril mit Synthesegas in Gegenwart des Kobaltcarbonyl-Polyvinylpyrrolidon-Katalysatorkomplexes als Hauptreaktion die Carbonylierung des Acrylnitrils zu den beiden isomeren Cyanpropionsäuremethylestern statt (Gesamtausbeute ca. 60 %), wohingegen die Hydroformylierung des Acrylnitrils (Bildung von 3-Cyanopropionaldehyd und 3-Cyanopropionaldehyd-dimethylacetal) nur als eine von mehreren Nebenreaktionen - Hydrierung der olefinischen Doppelbindung (Bildung von Propionitril), Michael-Addition des Methanols an die Doppelbindung (Bildung von 3-Methoxypropionitril) - unter den angegebenen Reaktionsbedingungen abläuft. Abgesehen davon führt die Verwendung des Lösungsmittels Methanol zu einer weiteren Nebenreaktion, nämlich der Acetalisierung des Hydroformylierungsprodukts, wodurch dessen Ausbeute zusätzlich erniedrigt wird. Die Lehre von US-A 4,235,744 stellt somit im wesentlichen auf herkömmliche Carbonylierungsreaktionen von Olefinen zu Carbonsäureestern ab und lehrt kein industriell anwendbares Hydroformylierungsverfahren. Zur Aufarbeitung des Reaktionsaustrags werden von US-A 4,235,744 zwei Methoden angegeben, nämlich fraktionierte Destillation und Lösungsmittelextraktion.

Schließlich ist aus US-A 3 594 425 ein Verfahren der Hydroformylierung bekannt, bei dem als Liganden phosphorfreie, niedermolekulare Polyamine, auch mit Alkylenoxiden derivatisierte, nieder-molekulare Polyamine, verwendet werden. Die im einzelnen beschriebenen Polyamine weisen alle ein Molekulargewicht kleiner 300 auf. Obgleich angegeben ist, daß die Polyamin-Liganden die Möglichkeit eröffnen, den Katalysatorkomplex mit dem Destillationssumpf in die Hydroformylierungsreaktion zurückzuführen, hat die Verwendung der dort beschriebenen Polyamine den Nachteil, daß sie relativ flüchtig sind und insbesondere bei der Hydroformylierung höherer Olefine zumindest teilweise in die Destillatfraktion des Reaktionsprodukts gelangen.Abgesehen davon sind die Katalysatorkomplexe mit diesen niedermolekularen Polyaminliganden bei starker thermischer Bealstung im Zuge der destillativen Aufarbeitung des Hydroformylierungsaustrags nicht stabil, so dass sich ein Teil des Katalysatormetalls abscheidet. Somit ist dieses Verfahren im großtechnischen Maßstab wirtschaftlich nicht durchführbar.

Es bestand daher die Aufgabe, Liganden zu finden, die ein industriell anwendbares Verfahren zur Hydroformylierung von Olefinen mit Katalysatorrückführung, das universell anwendbar ist und insbesondere bei der Hydroformylierung höherer Olefine eine vollständige Abtrennung und damit eine praktisch verlustfreie Rückführung des Katalysators und des Liganden gestattet und das nicht die anderen genannten Nachteile, wie Abscheidung metallischen Rhodiums oder destillative Ligandenverluste hat, ermöglichen.

Dementsprechend wurde ein Verfahren zur Herstellung von Aldehyden oder Aldehyden und Alkoholen durch Hydroformylierung von Olefinen in Gegenwart eines im Reaktionsgemisch homogen gelösten Komplexkatalysators, enthaltend ein Metall der Gruppe VIIIa des Periodensystems der Elemente und als Ligand eine phosphorfreie, mehrzähnige, zur Komplexbildung befähigte Stickstoffverbindung, bei Temperaturen von 50 bis 100°C und Drücken von 20 bis 1000 bar und Rückführung des Katalysatorkomplexes in die Hydroformylierungsreaktion gefunden, das dadurch gekennzeichnet ist, daß man
a) als Liganden derivatisierte und zur Komplexbildung befähigte Polyamine mit einem mittleren Molekulargewicht größer 1000 Dalton und mit mindestens 10 Stickstoffatomen verwendet,die sich zu nicht mehr als 1 g/l in Wasser bei Raumtemperatur lösen und aus dem Reaktionsgemisch nicht mit Wasser extrahiert werden können,
b) aus dem Reaktionsgemisch nach Beendigung der Hydroformylierungsreaktion und destillativer Abtrennung oder partieller destillativer Abtrennung der Aldehyde und Alkohole den im Destillationssumpf verbleibenden Katalysatorkomplex und überschüssigen Liganden vollständig oder zum Teil in die Hydroformylierung zurückführt und
c) kontinuierlich oder zumindest absatzweise zumindest einen Teil der Hochsieder aus dem Sumpf der Destillation des Reaktionsgemisches ausschleust.

Insbesondere wurde diese Aufgabe gelöst mit einem Verfahren zur Herstellung von Aldehyden oder Aldehyden und Alkoholen durch Hydroformylierung von Olefinen in Gegenwart eines im Reaktionsgemisch homogen gelösten Komplexkatalysators, enthaltend ein Metall der Gruppe VIIIa des Periodensystems der Elemente und als Ligand eine phosphorfreie, mehrzähnige, zur Komplexbildung befähigte Stickstoffverbindung, bei Temperaturen von 50 bis 100°C und Drükken von 20 bis 1000 bar und Rückführung des Katalysatorkomplexes in die Hydroformylierungsreaktion, wobei man
a) als Polyaminliganden Polyethylenimine bestehend im wesentlichen aus Einheiten der Formel I verwendet, 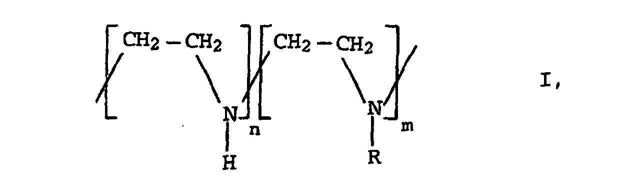 in der die Summe aus m + n mindestens 10 und das Verhältnis aus m/m+n 0,01 bis 1 beträgt und R gleiche oder verschiedene Alkyl-, Cycloalkyl-, Aryl-, Aralkyl-, Alkanoylgruppen mit bis zu 30 C-Atomen oder Hydroxyalkyl(poly)oxyalkylengruppen mit bis zu 500 Alkylenoxy-Einheiten bedeuten,
b) aus dem Reaktionsgemisch nach Beendigung der Hydroformylierungsreaktion und destillativer Abtrennung oder partieller destillativer Abtrennung der Aldehyde und Alkohole den im Destillationssumpf verbleibenden Katalysatorkomplex und überschüssigen Ligand vollständig oder zum Teil in die Hydroformylierung zurückführt und
c) kontinuierlich oder zumindest absatzweise zumindest einen Teil der Hochsieder aus dem Sumpf der Destillation des Reaktionsgemisches ausschleust,
   und hierbei
   - den Katalysator und überschüssigen Liganden mit einem Lösungsmittel, in dem der Katalysator und der Überschüssige Ligand unlöslich oder nahezu unlöslich ist, ausfällt und nur das Fällungsprodukt in die Hydroformylierung zurückführt, und/oder
   - den Katalysator und überschüssigen Liganden durch Ultrafiltration des Destillationssumpfes gewinnt und den an Hochsiedern abgereicherten und an Katalysator und Liganden angereicherten Retentatstrom in die Hydroformylierung zurückführt, und/oder
   - die im Destillationssumpf enthaltenen Hochsieder mittels Wasserdampfdestillation vom Katalysator und überschüssigem Liganden abtrennt und nur den nach der Wasserdampfdestillation erhaltenen, Katalysator und überschüssigen Liganden enthaltenden Destillationssumpf in die Hydroformylierungsreaktion zurückführt.

Der Begriff "derivatisierte, im wesentlichen nicht wasserlösliche und zur Komplexbildung befähigte Polyamine" im Sinne der vorliegenden Anmeldung bezieht sich auf polymere Polyamine, deren Aminogruppen durch Umsetzung mit z.B. Alkylierungs-, Arylierungs-, Amidierungs- oder Alkoxylierungsmitteln in so einem Maße alkyliert, aryliert, amidiert oder alkoxyliert worden sind oder aber auch durch chemische Abbaureaktionen, z.B. Hydrolyse oder Hydrogenolyse, aus vollständig an ihren Aminogruppen alkylierten, arylierten, amidierten oder alkoxylierten polymeren Ausgangspolyaminen solchermaßen durch Alkyl-, Aryl-, Alkanoyl- oder Alkoxylgruppen substituiert sind, daß diese polymeren Polyamine im wesentlichen wasserunlöslich, jedoch in der Reaktionsmischung der Hydroformylierungsreaktion löslich werden und mindestens noch ein Teil der Stickstoffatome der Aminogruppen zur Komplexbildung mit dem Metall der Gruppe VIIIa zur Verfügung steht. Solche derivatisierten Polyamine können z.B. aus Polyethylenimin oder Polyvinylamin oder Vorläuferverbindungen des Polyvinylamins, wie Polyvinylformamid, durch die vorgenannten Umsetzungen erzeugt werden. Bevorzugt werden im erfindungsgemäßen Verfahren derivatisierte Polyamine auf Basis von Polyethylenimin verwendet, insbesondere alkylierte, arylierte, amidierte oder alkoxylierte Polyethyleniminderivate.

Als erfindungsgemäß zu verwendende Liganden kommen insbesondere Polyethylenimine bestehend im wesentlichen aus Einheiten der Formel III in Betracht 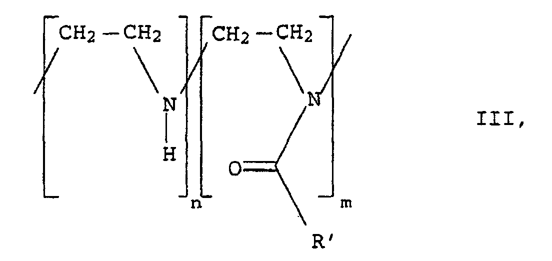 in der der Rest R' Alkyl mit 1 bis 30 C-Atomen, bevorzugt mit 1 bis 21 C-Atomen, bedeutet, die Summe aus n und m mindestens 10 und das Verhältnis aus m/m+n 0,01 bis 1 beträgt.

weitere in Betracht kommende Liganden sind Polyethylenimine bestehend im wesentlichen aus Einheiten der Formel IV 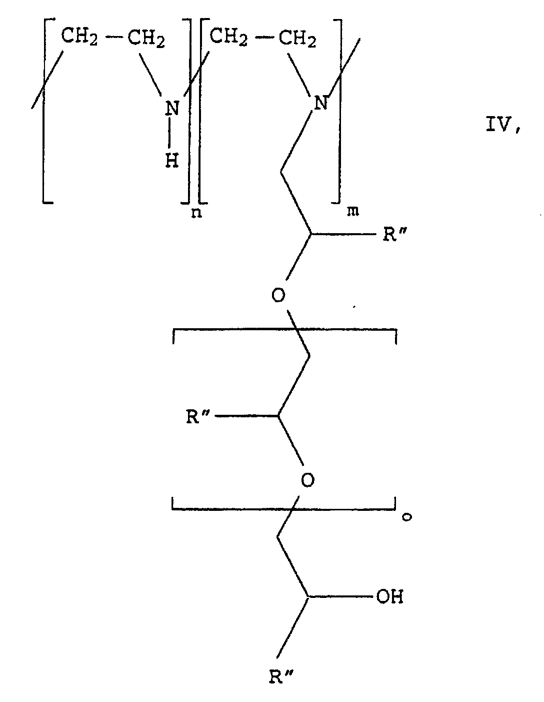 in der R" Wasserstoff oder niedermolekulares Alkyl, d.h. eine C₁bis C₁₂-, vorzugsweise C₁- bis C₄-Alkylgruppe, bedeutet, die Summe aus n und m mindestens 10 und das Verhältnis aus m/m+n 0,01 bis 1 beträgt und o einen Wert von 0 und 500 haben kann. Diese Verbindungen werden als alkoxylierte Polyethylenimine bezeichnet und sind in DE-A 44 35 688 und DE-A 22 27 546 beschrieben. Auf die Angaben dieser Vorliteratur, insbesondere in Bezug auf die Herstellung der Polyamine, wird ausdrucklich Bezug genommen und deren Angaben dazu sollen als hier inkorporiert gelten.

Als für das erfindungsgemäße geeignete Liganden sind außerdem Polyethylenimine zu nennen, die im wesentlichen aus Einheiten der Formel V 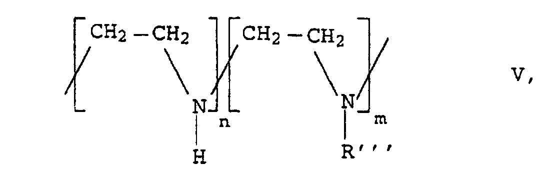 zusammengesetzt sind, worin R''' für gleiche oder verschiedene Gruppen, ausgewählt aus gegebenenfalls substituierten C₁- bis C₃₀-, vorzugsweise C₁- bis C₂₀-, insbesondere C₁- bis C₁₀-Alkyl-, C₃- bis C₈-Cycloalkyl-, C₆- bis C₁₂-Aryl, vorzugsweise Phenyl-, oder C7- bis C₁₂-Aralkyl, vorzugsweise Benzyl- oder Phenylethyl-Gruppen, steht und in der die Summe aus m + n mindestens 10 und das Verhältnis m/m + n 0,01 bis 1 beträgt. Unter den Polyethylenaminliganden der Formel V sind solche bevorzugt, in denen R''' eine geradkettige oder verzweigte Alkylgruppe ist. Die Polyethyleniminliganden der Formel V können auf konventionelle Weise z.B. aus Polyethylenimin mit den R''' entsprechenden Aldehyden oder Alkoholen durch reduktive Aminierung oder durch Umsetzung des Polyethylenimins mit den R''' entsprechenden Alkyl-, Cycloalkyl-, Aryl- oder Aralkylhalogeniden in Gegenwart einer Base hergestellt werden.

Das Molekulargewicht der erfindungsgemäß zu verwendenden derivatisierten Polyaminliganden gemäß Formeln I, III, IV und V beträgt mindestens 1000 Dalton, vorzugsweise mehr als 10000 Dalton. Hierbei handelt es sich um eine mittlere Molmasse (Gewichtsmittel Mw), da bei der Herstellung der Polyamine und weiterer Umsetzung wie üblich eine breite Molekulargewichtsverteilung eintritt.

Die erfindungsgemäß zu verwendenden Polyamin-Liganden sind im Hydroformylierungsmedium vollständig löslich, aber im wesentlichen wasserunlöslich. Dies bedeutet, daß z.B. nicht mehr als 1 g/l sich in Wasser bei Raumtemperatur lösen und sie aus dem Reaktionsgemisch nicht mit Wasser extrahiert werden können.

Im besonderen kommen Verbindungen bestehend im wesentlichen aus Einheiten der Formeln III bzw. IV in Betracht 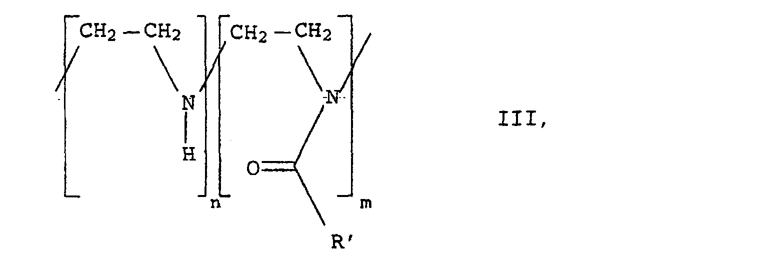 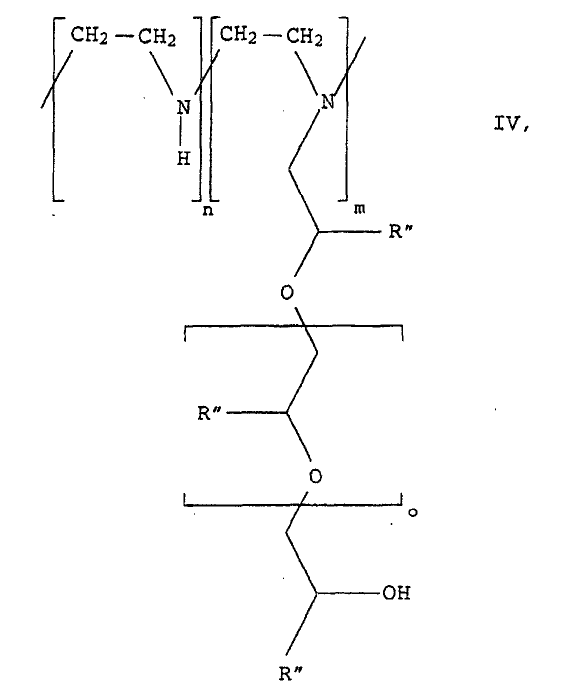 bei denen die Summe aus n + m mindestens 10 und das Verhältnis m/m+n 0,01 bis 1 beträgt o einen Wert bis zu 500 hat, R' eine Alkylgruppe mit 5 bis 30 C-Atomen und R" Wasserstoff oder die Methylgruppe bedeutet.

Im einzelnen kommen z.B. in Betracht:

Umsetzungsprodukte von Polyethylenimin (hergestellt durch Polymerisation von Aziridin) mit einem mittleren Molekulargewicht von 200 bis 2 Millionen Dalton, mit C₁- bis C₃₀-Carbonsäuren, bevorzugt C₅- bis C₃₀-Carbonsäuren, insbesondere mit aufgrund ihres günstigen Preises vorzugsweise mit aus natürlichen Quellen zur Verfügung stehenden Carbonsäuren, wie natürlich vorkommenden Fettsäuren oder deren Gemischen oder Naphthensäuren, beispielsweise mit Ameisensäure, Essigsäure, Propionsäure, Buttersäure, Valeriansäure, Capronsäure, Caprylsäure, Caprinsäure, Laurinsäure, Myristinsäure, Palmitinsäure, Stearinsäure, Arachinsäure, Behensäure, Ölsäure, Linolsäure, Linolensäure, Erucasäure usw., ferner mit aus technischen Quellen preiswert verfügbaren Carbonsäuren, wie 2-Ethylhexansäure sowie 2-Propylheptansäure, wobei der Amidierungsgrad 30 bis nahe 100 %, bezogen auf die amidierbaren Aminogruppen beträgt. Solche Polyethyleniminamide können z.B. nach dem Verfahren von DE-A 37 27 704 durch Umsetzung des Polyethylenimins mit den betreffenden Carbonsäureanhydriden oder Estern hergestellt werden.

Weiterhin Umsetzungsprodukte des vorgenannten Polyethylenimins mit 1 bis zu 500 Mol Ethylenoxid und/oder Propylenoxid und/oder 1,2-Butylenoxid pro Monomereinheit des Polyethylenimins, wie sie in DE-A 44 35 688 beschrieben sind.

Unter Hydroformylierungsbedingungen bilden sich aus Metallverbindungen, insbesondere Metallsalzen der Gruppe VIIIa des Periodensystems der Elemente und den erfindungsgemäß zu verwendenden Liganden in situ neue Hydroformylierungskomplexkatalysatoren der Formel II

L(M(CO)_{y}H_{z}) II,

in der L einen Liganden in Form eines derivatisierten, im wesentlichen nicht wasserlöslichen und zur Komplexbildung befähigten Polyamins mit mindestens 10 Stickstoffatomen und mit einem mittleren Molekulargewicht größer 1000 Dalton, vorzugsweise größer 10000 Dalton, insbesondere einen Liganden bestehend im wesentlichen aus Einheiten der Formel I 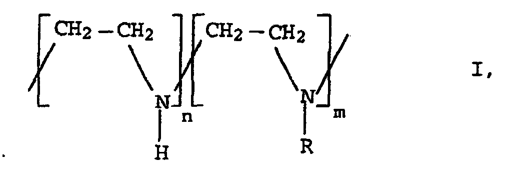 in der die Summe aus m + n mindestens 10 und das Verhältnis aus m/m+n 0,01 bis 1 beträgt und R gleiche oder verschiedene Alkyl-, Cycloalkyl-, Aryl-, Aralkyl-, Alkanoylreste mit bis zu 30 C-Atomen oder Hydroxyalkyl(poly)oxyalkylengruppen mit 1 bis zu 500 Alkylenoxy-Einheiten bedeuten, M ein Element der Gruppe VIIIa des Periodensystems der Elemente, insbesondere Rhodium oder Ruthenium, besonders bevorzugt Rhodium, y die Zahlen 1 bis 4 und z die Zahlen 0 bis 2 bedeutet.

Als zu hydroformylierende Olefine kommen beliebige Olefine, Olefine mit mehr als 3 C-Atomen z.B. solche mit 4 bis 24 C-Atomen, insbesondere Olefine von 4 bis 14 C-Atomen in Betracht.

Die Olefine können geradkettig oder verzweigt sein und können α-olefinische und/oder interne Doppelbindungen enthalten. Im einzelnen können z.B. Octen-1, Dodecen-1, Tetradecen-1, Trimer- und Tetramerpropylen, oder Dimer-, Trimer- und Tetramerbutylen im erfindungsgemäßen Verfahren eingesetzt werden. Ebenso können ungesättigte Oligomere anderer Olefine, z.B. Hexenoligomere, hydroformyliert werden, desgleichen Cooligomere verschiedener Olefine. Die aus diesen Olefinen gebildeten Aldehyde dienen als Vorstufen für die Herstellung von Weichmacheralkoholen und Tensiden, die daraus auf an sich bekannte Weise durch Hydrierung erzeugt werden können. Die zur Hydroformylierung eingesetzten Olefine können durch eine Vielzahl technischer Verfahren, wie sie beispielsweise in Weissermel, Arpe: Industrielle Organische Chemie, S. 67 - 86, Verlag Chemie, Weinheim, 1994, beschrieben sind, hergestellt werden.

Die Hydroformylierung erfolgt in an sich bekannter Weise bei Temperaturen von 50°C bis 200°C, vorzugsweise bei 70 bis 180°C und insbesondere bei 80 bis 170°C und Drücken von 5 bis 600 bar, vorzugsweise 10 bis 400 bar und insbesondere bei 20 bis 300 bar. Der Katalysator wird in der Regel in situ gebildet durch Zugabe einer das Metall der Gruppe VIIIa des Periodensystems der Elemente enthaltenden Verbindung und einem Überschuß z.B. einem 2 bis 1000 molaren Überschuß, vorzugsweise einem 4 bis 500-fachen und insbesondere einem 10 bis 100-fachen molaren Überschuß des Liganden, vorzugsweise dem der Formel I, berechnet auf der Basis der Monomereinheiten des Polyamins, so daß sich unter den Reaktionsbedingungen die Komplexkatalysatoren der Formel II bilden. Da die erfindungsgemäß verwendeten Katalysatoren außer ihrer Hydroformylierungsaktivität auch eine gewisse Hydrieraktivität haben, entstehen bei als Wertprodukte zusätzlich zu den Aldehyden auch die diesen Aldehyden entsprechenden Alkohole.

Im übrigen wird auf einschlägige Literatur über die Hydroformylierung z.B. Falbe s.o. verwiesen.

Der Austrag aus der Hydroformylierungsstufe wird vor seiner destillativen Aufarbeitung entspannt. Dabei wird nicht umgesetztes Synthesegas freigesetzt, das in die Hydroformylierung zurückgeführt werden kann. Entsprechendes gilt für das bei der Entspannung in die Gasphase übergegangene, nicht umgesetzte Olefin, das, gegebenenfalls nach destillativer Abtrennung darin enthaltener, inerter Kohlenwasserstoffe, ebenfalls in die Hydroformylierung zurückgeführt werden kann.. Die Destillation des entspannten Hydroformylierungsaustrags wird im allgemeinen bei Drücken von 0,1 bis 1000 mbar absolut, vorzugsweise von 1 bis 500 mbar und besonders bevorzugt von 10 bis 200 mbar durchgeführt.

Die Temperatur, die in der Destillation eingestellt werden muß, ist abhängig von der Art des Hydroformylierungsprodukts und der verwendeten Destillationsapparatur. Für das erfindungsgemäße Verfahren können im allgemeinen beliebige Destillationsapparaturen verwendet werden. Bevorzugt werden jedoch Apparate eingesetzt, die niedrige Investitionskosten verursachen und eine möglichst niedrige Destillationstemperatur erlauben, wie Dünnschichtverdampfer, Wischblattverdampfer oder Fallfilmverdampfer, da mit höherer Temperatur die gebildeten Aldehyde im Reaktionsaustrag Folgereaktionen wie Aldolkondensationen eingehen können. Da diese Destillation im wesentlichen der Abtrennung der Hydroformylierungsprodukte Aldehyd und Alkohol und gegebenenfalls noch vorhandener anderer Leichtsieder, wie nicht umgesetztem Olefin und Inerten, von hochsiedenden Kondensationsprodukten der Aldehyde, sogenannten Hochsiedern, und dem Katalysator und überschüssigem Liganden dient, kann es zweckmäßig sein, die so abgetrennten Hydroformylierungsprodukte und gegebenenfalls Olefine und Inerte, einer weiteren destillativen Reinigung, die auf herkömmliche Weise durchgeführt werden kann, zu unterziehen.

Ein wesentliches Kombinationsmerkmal des erfindungsgemäßen Verfahrens ist die Rückführung des Komplexkatalysators und des überschüssigen Liganden aus dem Destillationsrückstand des Reaktionsgemisches. Dabei kann entweder
a) der Destillationssumpf, der den Katalysator und überschüssigen Ligand enthält, insgesamt zurückgeführt werden, oder
b) der Katalysator und überschüssiger Ligand mit einem Lösungsmittel, in dem der Katalysator und der überschüssige Ligand unlöslich oder nahezu unlöslich ist, ausgefällt und nur das Fällungsprodukt zurückgeführt werden, oder
c) der Katalysator und überschüssiger Ligand durch Ultrafiltration des Destillationssumpfes gewonnen und das Retentat zurückgeführt werden oder
d) die im Destillationssumpf enthaltenen Hochsieder mittels Wasserdampfdestillation vom Katalysator und überschüssigem Liganden abgetrennt werden und nur der nach der Wasserdampfdestillation erhaltene, Katalysator und überschüssigen Liganden enthaltene Destillationssumpf in die Hydroformylierungsreaktion zurückgeführt wird, wobei im Falle (a) zur Vermeidung einer Anreicherung von Hochsiedern im Kreislauf eine Kombination mit den Methoden b) und/oder c) und/oder d) vorteilhaft ist.

Es versteht sich für den Fachmann von selbst, daß alternativ zu den Methoden b), c) und d) zwecks Vermeidung einer Aufpegelung von Hochsiedern im Reaktionsgemisch der Hydroformylierung auch ein Teil des Destillationssumpfes aus dem Verfahren von Zeit zu Zeit ausgeschleust werden und der weiteren Aufarbeitung zur Rückgewinnung des Gruppe VIIIa Metalls und gewünschtenfalls des verwendeten Liganden zugeführt werden kann. Bei einer solchen Vorgehensweise ist es selbstverständlich, daß eine der ausgeschleusten Menge an Gruppe VIIIa Metall und Ligand entsprechende Menge dieser Verbindungen durch Zufuhr dieser Verbindungen in die Hydroformylierungsreaktion ergänzt werden muß.

### Methode a)

Man verwendet bevorzugt solche erfindungsgemäße Liganden, die bei 25°C einen Dampfdruck von weniger als 10⁻⁵, vorzugsweise weniger als 10⁻⁶ mbar aufweisen. Dies bedeutet, daß der Polyamin-Ligand einen so hohen Siedepunkt hat, daß bei der Destillation des Reaktionsaustrages der gesamte Ligand-Gruppe-VIIIa-Metall-Komplex und der gesamte nicht zur Komplexbildung benötigte Ligand im Sumpf der Destillation verbleiben und der Sumpf aus dieser Destillation zusammen mit frischem Olefin in die Reaktion zurückgeführt werden kann.

Dieses Verfahren führt überraschenderweise zu ausgezeichneten Ergebnissen bei der Hydroformylierung mit destillativer Aufarbeitung des Reaktionsaustrages, da die zu verwendenden Liganden sehr gute Stabilisatoren für den thermolabilen Katalysator sind und aufgrund ihres niedrigen Dampfdruckes quantitativ im Destillationssumpf verbleiben und somit Verluste des Liganden als auch der Metallkomponente des Katalysators weitestgehend vermieden werden können, selbst wenn eine sehr kostengünstige Destillation mit einer niedrigen Zahl von theoretischen Böden wie z.B. Dünnschichtverdampfung oder Fallfilmverdampfung eingesezt wird.

Da bei dieser Variante des Verfahrens sämtliche hochsiedende Nebenprodukte wieder in die Reaktion gelangen, tritt eine gewisse Aufpegelung der Hochsieder ein und es wird erforderlich kontinuierlich oder absatzweise eine Ausschleusung von Hochsiedern vorzunehmen. Dies kann z.B. dadurch geschehen, daß man nach den unten beschriebenen Varianten b), (c) und d) zumindest zeitweise eine Abtrennung des Katalysatorkomplexes und des überschüssigen Liganden aus dem Destillationssumpf vornimmt und den überwiegend Hochsieder enthaltenden Rest ausschleust.

### Methode b)

Wesentlich für diese Methode der Fällung des Katalysatorkomplexes und des überschüssigen Liganden ist eine Lösungsmittel, das sich mit den organischen Bestandteilen des Destillationssumpfes der Reaktionsaustrag in einem weiten Bereich mischbar ist, in dem jedoch der Katalysatorkomplex und der Ligand unlöslich oder nahezu unlöslich ist, so daß es möglich wird, durch Wahl der Art und Menge des Lösungsmittels den Katalysatorkomplex und den Ligand auszufällen, die nach Abtrennung durch Dekantation oder Filtration in die Hydroformylierung zurückgeführt werden kann, wobei dies bevorzugt in Form einer Lösung z.B. gelöst in dem der Reaktion zuzuführenden Olefin geschieht.

Als Lösungsmittel kommt eine große Zahl von polaren Lösungsmitteln, die vor allem 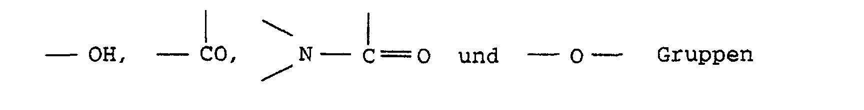 aufweisen, also Alkohole, Ketone, Amido oder Ether, vorzugsweise Aceton, Tetrahydrofuran, Dimethylformamid, Dimethylacetamid, N-Alkylpyrrolidone, Glykole, Methanol, Ethanol, n-Propanol, Isopropanol sowie beliebige Mischungen dieser Lösungsmittel oder Mischungen dieser Lösungsmittel mit Wasser in Betracht.

Die Art und Menge des anzuwendenden Lösungsmittel kann der Fachmann durch wenige Handversuche im einzelnen ermitteln. Im allgemeinen wird die Menge des Lösungsmittels möglichst niedrig gehalten, damit der Wiedergewinnungsaufwand möglichst gering ist. Demgemäß werden in der Regel, bezogen auf das Volumen des Destillationssumpfes, die 1 bis 50, vorzugsweise die 3 bis 15-fache Menge benötigt.

### Methode c)

Infolge des großen Unterschieds der Molekulargewichte des Katalysatorkomplexes und überschüssigen Liganden einerseits und der im Destillationssumpf verbleibenden Hochsieder andererseits ist es auch möglich, Katalysatorkomplex und Ligand durch Ultrafiltration von den Hochsiedern abzutrennen. Dazu beträgt das mittlere Molekulargewicht des Liganden mehr als 1000 Dalton, vorzugsweise mehr als 10000 Dalton.

Das Verfahren kann kontinuierlich oder diskontinuierlich betrieben werden.

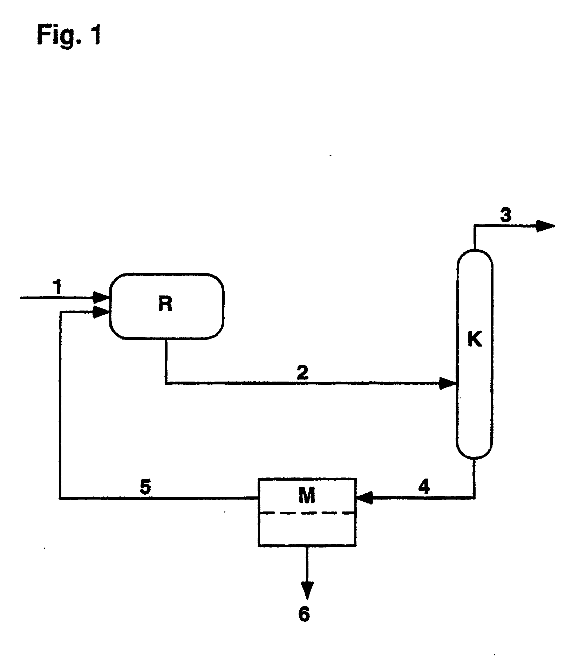

Bei der kontinuierlichen Arbeitsweise (Figur 1) werden die Edukte 1 in Anwesenheit vom Katalysator und Liganden in dem Reaktor R umgesetzt. Der Reaktoraustrag 2 wird in einer Destillationsapparatur K in einen Destillatstrom 3, der die Oxoprodukte enthält, und in einen Rückstandstrom 4 getrennt. Der katalysatorhaltige Sumpf 4 wird kontinuierlich einer Membranfiltrationsanlage M zugeleitet. In dieser Membrananlage wird der Sumpf, der Hochsieder (oder ein Gemisch Hochsieder, Edukte und Oxoprodukte), Katalysator und Liganden enthält, aufgearbeitet. Die Hochsieder (und gegebenenfalls Edukte und Oxoprodukte) permeieren durch die Membran. Der an Hochsieder (und gegebenenfalls an Edukten und Oxoprodukten) abgereicherte und an Katalysator und Liganden angereicherte Retentatstrom 5 wird in die Hydroformylierung zurückgeführt.

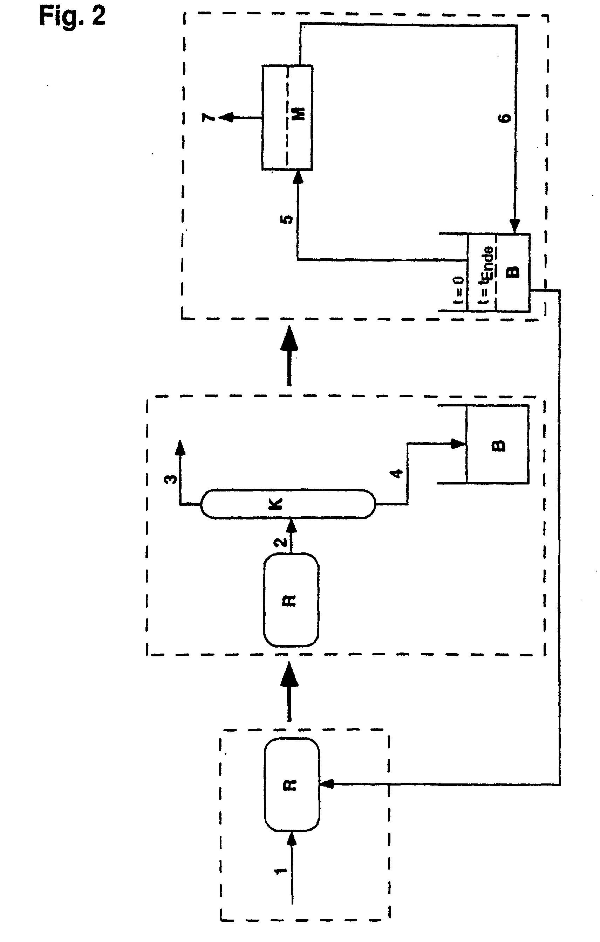

Bei der diskontinuierlichen Fahrweise (Figur 2) werden gleichermaßen die Edukte 1 in Anwesenheit vom Katalysator und Liganden in dem Reaktor R umgesetzt. Am Ende der Reaktion wird der Reaktoraustrag in einer Destillationsapparatur K in einen Destillatstrom 3, der die Oxoprodukte enthält, und in einen Rückstandstrom 4 getrennt. Dieser katalysatorhaltige Sumpf aus der Destillation wird in dem Behälter B gesammelt. Am Ende der Destillation kann der Behälterinhalt als Batch in der Membranfiltrationsanlage M aufgearbeitet werden. Der an Hochsieder (und gegebenenfalls an Edukten und Oxoprodukten) abgereicherte und an Katalysator und Liganden angereicherte Destillationssumpf wird am Ende der Ultrafiltration in den Reaktor für die nächste Charge der Hydroformylierung zurückgeführt.

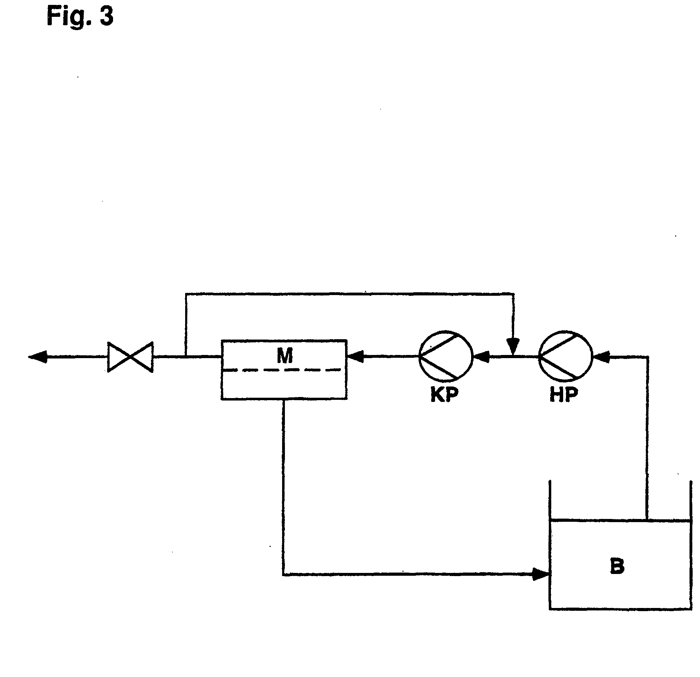

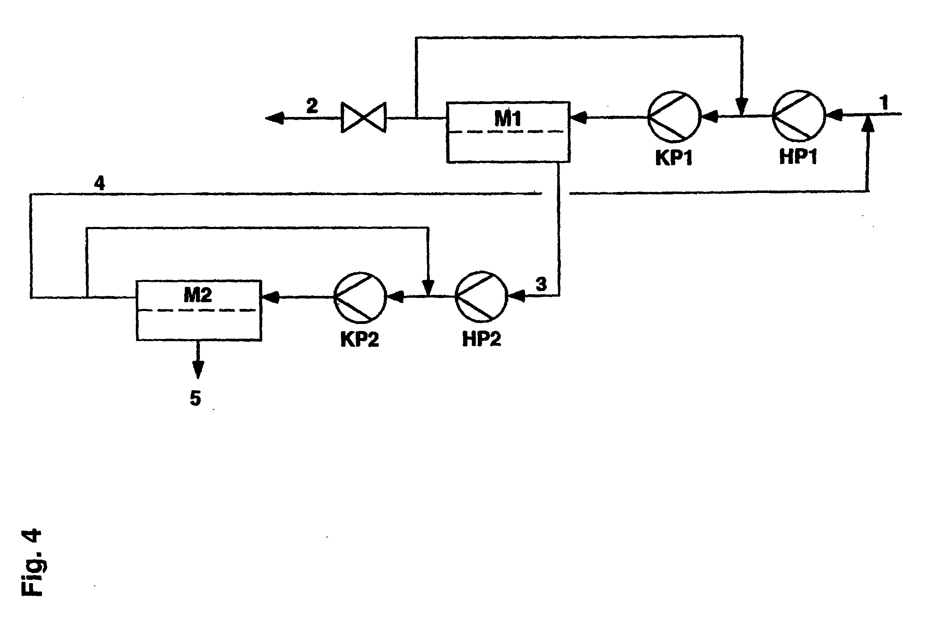

Die Ultrafiltration kann einstufig (Figur 3) oder mehrstufig (vorzugsweise zweistufig gemäß Figur 4) betrieben werden. In jeder Stufe wird die Feedlösung in einer Druckpumpe HP auf Filtrationsdruck gebracht; in einer zweiten Pumpe KP wird dann durch Rückführung eines Teils des Retentatstroms die Überströmung, d.h. Benetzung, der Membran sichergestellt. In der mehrstufigen Variante wird der Permeatstrom einer Stufe der nachgeschalteten Stufe zugeleitet und der Retentatstrom dieser nachgeschalteten Stufe der vorherigen Stufe zugeleitet. Durch diese Aufarbeitung des Permeats ist eine bessere Rückhaltung des Katalysators und des Liganden erreichbar.

Im Fall einer mehrstufigen Anlage können die verschiedenen Stufen mit der gleichen Membran oder mit unterschiedlichen Membranen ausgerüstet sein.

Die Ultrafiltration wird bei Transmembrandrücken von 0,5 bis 20 bar, vorzugsweise 2 bis 12 bar und bei Temperaturen bis 200°C je nach Membranmaterial betrieben. Die Überströmungsgeschwindigkeit in dem Modul beträgt 1 bis 10, vorzugsweise 1 bis 4 m/s. Die Katalysatorkonzentration in der Feedlösung zur Membran beträgt 5 bis 2000 ppm, die Polymerkonzentration, also die Konzentration an freiem Polyamin-Ligand, beträgt 1 bis 12 Gew.-%.

Für die Ultrafiltration kommen alle Membranen in Betracht, die im System stabil sind. Die Trenngrenze der Membranen beträgt 500 bis 200 000 Dalton. Für das Membranmaterial kommen Polymere (1), keramische Materialien (2), Glas, Metall oder Keramik auf Metall (3) in Betracht. In der folgenden Tabelle sind Beispiele von solchen Membranen zusammengestellt.

| Typ | Bezeichnung | Material | Trenngrenze | Hersteller |
|---|---|---|---|---|
| (1) | MPF-U20-S | Polysulfon | 20 000 Dalton | Membran Products Kiryat Weizmar |
| (2) | K00X1040 | ZiO₂ auf Al₂O₃-TiO₂ | 15 000 Dalton | Tech-Sep |
| (2) | | TiO₂ | 5 000 bis 10 000 Dalton | Inocermic Gesellschaft für Innovative Keramik mbH |
| (2) | | TiO₂ | 5 000 Dalton | Société des Céramiques Techniques |
| (3) | | TiO₂ auf Edelstahl | | Graver Chemical Company |

### Methode d)

Eine weitere Möglichkeit zur Ausschleusung von hochsiedenden Kondensationsprodukten der Aldehyde besteht darin, diese aus dem Sumpf der Destillation mittels Wasserdampfdestillation abzutrennen. Die Wasserdampfdestillation des Destillationssumpfes kann diskontinuierlich, also absatzweise, oder kontinuierlich erfolgen, wobei die Wasserdampfdestillation in der Destillationsapparatur selbst oder einer separaten Vorrichtung zur Wasserdampfdestillation vorgenommen werden kann. Beispielsweise kann bei der diskontinuierlichen Ausgestaltung des Verfahrens der Destillationssumpf vor seiner Rückführung in die Hydroformylierung durch Durchleiten von Wasserdampf ganz oder teilweise von hochsiedenden Kondensationsprodukten befreit werden oder der Destillationssumpf je nach Hochsiederanfall von Zeit zu Zeit in einer separaten Apparatur einer Wasserdampfdestillation unterzogen werden. Die kontinuierliche Ausgestaltung dieser Methode kann z.B. so erfolgen, daß der Destillationssumpf oder ein Teil des Destillationssumpfes vor seiner Rückführung in die Hydroformylierung kontinuierlich einer Wasserdampfdestillationsapparatur zugeführt und darin ganz oder teilweise von Hochsiedern befreit wird. Ebenso ist es möglich, die destillative Aufarbeitung des Hydroformylierungsaustrags von vornherein kontinuierlich in Gegenwart von Wasserdampf zu betreiben, um gleichzeitig mit dem Aldehyd und dem Alkohol auch die Hochsieder vom Katalysator und dem Liganden abzutrennen.

Es versteht sich von selbst, daß bei einer solchen Vorgehensweise in einer nachfolgenden Fraktionier- oder Destillationseinrichtung die Wertprodukte von den Hochsiedern und gegebenenfalls von Wasser geschieden werden müssen.

Die Wasserdampfdestillation erfolgt im allgemeinen auf herkömmliche Weise durch Einleiten von Wasserdampf in den Hochsieder enthaltenden Destillationssumpf und nachfolgende Kondensation des Wasserdampfdestillates. Vorteilhaft wird dabei der Wasserdampf so durch den Destillationssumpf geleitet, daß er nicht im Destillationssumpf kondensiert. Dies kann durch die Wahl der Druck- und/oder Temperaturbedingungen, unter denen die Wasserdampfdestillation durchgeführt wird, bewirkt werden. Dabei kann sowohl verminderter Druck angewendet werden oder bei Verwendung überhitzten Wasserdampfes auch erhöhter Druck. Im allgemeinen wird die Wasserdampfdestillation bei einer Temperatur von 80 bis 200°C und bei einem Druck von 1 mbar bis 10 bar, vorzugsweise von 5 mbar bis 5 bar, vorgenommen. Dabei wird der Wasserdampf bezüglich der im Sumpf enthaltenen hochsiedenden Kondensationsprodukte der Aldehyde (Hochsieder) im allgemeinen in einem Gewichtsverhältnis Wasserdampf : Hochsieder von 10 : 1 bis 1 : 10 durch den Destillationssumpf geleitet. Nach Beendigung der Wasserdampfdestillation kann der so ganz oder teilweise von Hochsiedern befreite, Katalysator und überschüssigen Liganden enthaltende Destillationssumpf in die Hydroformylierung zurückgeführt werden.

Wie bereits bei der Methode (a) erwähnt, kann es zweckmäßig sein, die Methoden a) und b) bzw. a) und c) bzw. a) und d) zu kombinieren.

In allen Fällen führt das erfindungsgemäße Verfahren zu gegenüber dem Stand der Technik verbesserten Ergebnissen, da es nun mit den erfindungsgemäß zu verwendenden Liganden gelingt, sowohl das sehr zersetzungsempfindliche "nackte" Rhodium zu stabilisieren als auch den Katalysatorkomplex und überschüssigen Ligand in großtechnisch verwertbarer weise in die Hydroformylierungsreaktion zurückzuführen.

### Beispiele

### (A) Herstellung der derivatisierten Polyaminliganden

### Beispiel 1

Allgemeines Beispiel für die Ethoxylierung eines Polyamins, hier beschrieben für ein Polyethylenimin mit einem mittleren Molekulargewicht von 5000.

### Beispiel 1a)

### Monoalkoxylierung eines Polyethylenimins in Wasser (erste Stufe)

In einen Autoklaven wurden 43 g eines Polyethylenimins mit einem gewichtsgemittelten Molekulargewicht von ca. 5000 zusammen mit 43 g Wasser vorgelegt. Unter Rühren wurde dann bei einer Temperatur von 90 bis 100°C und einem Druck von max. 4 bar 44 g Ethylenoxid während 30 min zugegeben. Man rührte 1 h bei dieser Temperatur nach und fügte 20 g 50 Gew.-%ige wäßrige KOH-Lösung zu. Bei vermindertem Druck (bis ca. 10 mbar) wurde das Wasser vollständig abdestilliert, wobei man während 5 h die Temperatur bis auf 120°C erhöhte.

### Beispiel 1b

### Weitere Oxalkylierung (zweite Stufe)

In einem Autoklaven wurde die Vorstufe aus Beispiel 1a bei einer Temperatur von 130 - 140°C und einem Druck bis ca. 4,5 bar mit ca. 480 g Ethylenoxid umgesetzt.

### Beispiel 2

Allgemeines Beispiel für die Propoxylierung eines Polyamins, hier beschrieben für ein Polyethylenimin mit einem mittleren Molekulargewicht (Gewichtsmittel) von 20000.

### Beispiel 2a

### Monoalkoxylierung eines Polyethylenimins in Wasser (erste Stufe) und azeotrope Destillation des Wassers nach Zugabe von Xylol

In einen Autoklaven wurden 43 g eines Polyethylenimins mit einem gewichtsgemittelten Molekulargewicht von ca. 20000 zusammen mit 43 g Wasser vorgelegt. Unter Rühren wurde dann bei einer Temperatur von 90 bis 100°C und einem Druck von max. 4 bar 58 g Propylenoxid während 30 min zugegeben. Man rührte 1 h bei dieser Temperatur nach, kühlte auf ca. 80°C ab, fügte 20 g 50 Gew.-%ige wäßrige KOH-Lösung und 100 g Xylol zu und trennte sämtliches Wasser durch azeotrope Destillation ab.

### Beispiel 2b

### Weitere Oxalkylierung (zweite Stufe) in Gegenwart von Xylol

In einem Autoklaven wurde die Vorstufe aus Beispiel 2a bei einer Temperatur von 130 - 140°C und einem Druck bis ca. 4,5 bar mit 4234 g Propylenoxid umgesetzt.

### Beispiel 3

### Synthese eines zu 50 mol-% (bezogen auf amidierbare Aminogruppen) mit Stearinsäure amidiertem Polyethylenimin

In einem 4 Liter fassenden Kolben, der mit einem Rührer, Thermometer und einer Einrichtung zum Arbeiten unter Stickstoff ausgestattet war, wurden 258 g Polyethylenimin eines mittleren Molekulargewichts (M_{w}) von ca. 25000 g/mol unter einem Stickstoffstrom auf eine Temperatur von 140°C erhitzt. Innerhalb von 30 min gab man portionsweise 853,5 g Stearinsäure hinzu. Die Reaktionstemperatur wurde auf 180°C erhöht, wobei die Mischung sich gelb färbte und aufschäumte. Das entstehende Wasser wurde kontinuierlich abdestilliert. Nach 18 h ließ man abkühlen und erhielt 1059 g eines leicht gelben Feststoffs. Das Reaktionsprodukt war klar löslich in Xylol, unlöslich in Wasser. Durch Titration mit Tetrabutylammoniumhydroxid in Xylol konnte kein Restsäuregehalt ermittelt werden.

### Beispiel 4

### Synthese eines mit 60 mol-% (bez. auf amidierbare Aminogruppen) Laurinsäure amidiertem Polyethylenimins

In einem 2 Liter fassenden Vierhalskolben mit Rührer, Tropftrichter, Temperaturmessung und einer Destillationsbrücke schmolz man 297 g (1,48 mol) Laurinsäure unter einem leichten Stickstoffstrom bei 100°C auf. Man erhitzte die Schmelze auf 150°C und fügte über den Tropftrichter 212,5 g einer 50 %igen Lösung eines Polyethylenimins (2,47 mol) eines mittleren Molekulargewichts (Mw) von 460000 hinzu. Die Zutropfgeschwindigkeit wurde dabei so gewählt, daß die Temperatur zwischen 150 und 155°C gehalten wurde und das zugeführte Wasser abdestillierte. Anschließend erhitzte man für 15 h auf 180°C, wobei das Reaktionswasser der Amidierung abdestilliert wurde. Die Schmelze wurde auf 100°C abgekühlt, dem Reaktor entnommen und bei Raumtemperatur zerkleinert. Man erhielt 364 g eines gelben Feststoffs.

Das Reaktionsprodukt war klar löslich in Xylol und Tetrahydrofuran, unlöslich in Wasser. Durch Titration mit Tetrabutylammoniumhydroxyd in Xylol konnte kein Restsäuregehalt ermittelt werden.

### (B) Hydroformylierung

### Beispiel 5

### Hydroformylierung von Dimerbuten mit einem amidiertes Polyethylenimin (aus Beispiel 3) enthaltenden Rh-Katalysator

5,01 mg Rhodiumbiscarbonylacetylacetonat (0,0194 mmol), 1,0 g amidiertes Polyethylenimin aus Beispiel 3, 100 g Dimerbuten (0,891 mmol) und CO/H₂ wurden auf 150°C erhitzt. Mittels CO/H₂ 1:1 wurde der gewünschte Druck von 280 bar eingestellt. Nach 3,5 Stunden wurde der Autoklav abgekühlt, entspannt und entleert. Die Analyse des Reaktionsgemisches mittels Gaschromatographie mit internem Standard und Korrekturfaktoren ergab einen Umsatz von Dimerbuten von 98 %, eine Ausbeute an Nonanalen von 85,6 % und eine Ausbeute an Nonanolen von 9,5 %.

### Beispiel 6

### Hydroformylierung von Octen-1 mit einem amidiertes Polyethylenimin (aus Beispiel 3) enthaltendem Rh-Katalysator

12,53 mg Rhodiumbiscarbonylacetylacetonat (0,0485 mmol), 1,0 g amidiertes Polyethylenimin, 99 g Octen-1 (0,882 mol) und CO/H₂ wurden auf 100°C erhitzt. Mittels CO/H₂ 1:1 wurde auf einen Druck von 280 bar eingestellt. Nach 5 Stunden wurde der Autoklav abgekühlt, entspannt und entleert. Die Analyse des Reaktionsgemisches mittels GC - mit internem Standard und Korrekturfaktoren - ergab einen Umsatz von Octen-1 von 99,7 %, eine Ausbeute an Nonanalen von 99,3 % (n-Anteil 53,6 %) und eine Selektivität für die Bildung von internen Olefinen von 0,18 %.

### Beispiel 7

### Hydroformylierung von Dodecen-1 mit einem amidiertes Polyethylenimin (aus Beispiel 3) enthaltendem Rh-Katalysator

12,53 mg Rhodiumbiscarbonylacetylacetonat (0,0485 mmol), 1,0 g amidiertes Polyethylenimin, 99 g Dodecen-1 (0,588 mol) und CO/H₂ wurden auf 100°C erhitzt. Mittels CO/H₂ 1:1 wurde auf einen Druck von 280 bar eingestellt. Nach 5 Stunden wurde der Autoklav abgekühlt, entspannt und entleert. Die Analyse des Reaktionsgemisches mittels GC - mit internem Standard und Korrekturfaktoren - ergab einen Umsatz von Dodecen-1 von 98,4 %, eine Ausbeute an Tridecanalen von 97,1 %, eine Ausbeute an Tridecanolen von 0,5 %, eine Selektivität an Tridecanalen von 98,6 % (n-Anteil 52,7 %) und eine Selektivität zu Tridecanolen von 0,5 %.

### Beispiel 8

### Hydroformylierung einer Mischung aus Dodecen-1 und Tetradecen-1 mit einem amidiertes Polyethylenimin (aus Beispiel 4) enthaltenden Rh-Katalysator

5,01 mg Rhodiumbiscarbonylacetylacetonat (0,0194 mmol), 1,0 g amidiertes Polyethylenimin, 100 g einer Mischung aus Dodecen-1 und Tetradecen-1, Mischungsverhältnis 2/1 und CO/H₂ wurden auf 100°C erhitzt. Mittels CO/H₂ 1:1 wurde der gewünschte Druck von 90 bar eingestellt. Nach 5 Stunden wurde der Autoklav abgekühlt, entspannt und entleert. Die Analyse des Reaktionsgemisches mittels GC - Flächenprozent-Auswertung - ergab einen Umsatz von Dodecen-1 von 99 %, einen Umsatz von Tetradecen-1 von 99 % und eine Gesamtausbeute an Alkoholen und Aldehyden von 97,5 %.

### Beispiel 9

### Hydroformylierung von Dodecen-N (Trimerbuten) mit einem amidiertes Polyethylenimin (aus Beispiel 3) enthaltendem Rh-Katalysator

12,53 mg Rhodiumbiscarbonylacetylacetonat (0,0485 mmol), 1,0 g amidiertes Polyethylenimin, 99 g Dodecen-N (0,588 mol) und CO/H₂ wurden auf 150°C erhitzt. Mittels CO/H₂ 1:1 wurde ein Druck von 280 bar eingestellt. Nach 5 Stunden wurde der Autoklav abgekühlt, entspannt und entleert. Die Analyse des Reaktionsgemisches mittels GC - Flächen-% Bestimmung - ergab einen Umsatz von Dodecen-N von 93,8 %, eine Ausbeute an Tridecanalen von 92,4 % und eine Ausbeute an Tridecanolen von 0,7 %.

### Beispiel 10

### Hydroformylierung von internen n-Dodecenen mit einem amidiertes Polyethylenimin (aus Beispiel 3) enthaltendem Rh-Katalysator

12,53 mg Rhodiumbiscarbonylacetylacetonat (0,0485 mmol), 1,0 g amidiertes Polyethylenimin, 99 g Dodecene mit internen Doppelbindungen (0,588 mol) und CO/H₂ wurden auf 130°C erhitzt. Mittel CO/H₂ 1:1 wurde ein Druck von 280 bar eingestellt. Nach 5 Stunden wurde der Autoklav abgekühlt, entspannt und entleert. Die Analyse des Reaktionsgemisches mittels GC - mit internem Standard und Korrekturfaktoren - ergab einen Umsatz von internen Dodecenen von 98,2 %, eine Ausbeute an Tridecanalen von 93,9 % und eine Ausbeute an Tridecanolen von 2,7 %.

### Beispiel 11

### Hydroformylierung von Dimerbuten mit einem propoxyliertes Polyethylenimin (aus Beispiel 2) enthaltendem Rh-Katalysator

5 mg Rhodiumbiscarbonylacetylacetonat (0,0194 mmol), 9,7 g propoxyliertes Polyethylenimin, 95 g Dimerbuten (0,85 mol), 5 g Palatinol® C (Dibutylphthalat) und CO/H₂ wurden auf 150°C erhitzt. Mittels CO/H₂ 1:1 wurde ein Druck von 280 bar eingestellt. Nach 5 Stunden wurde der Autoklav abgekühlt, entspannt und entleert. Die Analyse des Reaktionsgemisches mittels GC - mit internem Standard und Korrekturfaktoren - ergab einen Umsatz von 87,7 %, eine Ausbeute ans Nonanalen von 78,1 % und eine Ausbeute an Nonanolen von 4,1 %.

### Beispiel 12

### Hydroformylierung von Dimerbuten mit einem propoxyliertes Polyethylenimin (aus Beispiel 2) enthaltendem Rh-Katalysator

5 mg Rhodiumbiscarbonylacetylacetonat (0,0194 mmol), 9,7 g propoxyliertes Polyethylenimin, 95 g Dimerbuten (0,85 mol), 5 g Palatinol® C (Dibutylphthalat) und CO/H₂ wurden auf 130°C erhitzt. Mittels CO/H₂ 1:1 wurde auf einen Druck von 280 bar eingestellt. Nach 5 Stunden wurde der Autoklav abgekühlt, entspannt und entleert. Die Analyse des Reaktionsgemisches mittels GC - mit internem Standard und Korrekturfaktoren - ergab einen Umsatz von 78,1 %, eine Ausbeute an Nonanalen von 72,0 %, und eine Ausbeute an Nonanolen 0,9 %.

### Beispiel 13

### Hydroformylierung von Isobuten mit einem amidiertes Polyethylenimin (aus Beispiel 3) enthaltendem Rh-Katalysator

7,1 mg Rhodiumbiscarbonylacetylacetonat (0,074 mmol), 0,385 g amidiertes Polyethylenimin, 47,5 g Texanol®, 31,5 g Isobuten (0,56 mol) und CO/H₂ wurden auf 130°C erhitzt. Mittels CO/H₂ 1:1 wurde ein Druck von 280 bar eingestellt. Nach 3 Stunden wurde der Autoklav abgekühlt, entspannt und entleert. Die Analyse des Reaktionsgemisches mittels GC - mit internem Standard und Korrekturfaktoren - ergab einen Umsatz an Isobuten von 100 %, eine Ausbeute an 3-Methylbutanol von 96 % und eine Ausbeute an Pivalaldehyd von 0,2 %.

### (C) Katalysatorabtrennung und Rückführung

### Beispiel 14

Diskontinuierliche Hydroformylierung, Destillation und Ausfällung von Katalysator und von überschüssigem Ligand und Rückführung in die Hydroformylierung.

Beispiel 5 wurde mit der 10-fachen Einsatzmenge wiederholt. Nach beendeter Umsetzung wurde der Reaktionsaustrag bei 150°C und 10 mbar destilliert. Bei einer Rückstandsmenge von 100 g wurde die Destillation abgebrochen. Die Konzentration des Destillationsrückstandes an amidiertem Polyethylenimin betrug 12,7 %, die Rh-Konzentration betrug 249 ppm.

Der Destillationssumpf wurde mit der 10-fachen Masse Aceton und mit der 0,2-fachen Masse Wasser versetzt. Die Mischung wurde 30 min gerührt und 10 h bei 7°C gehalten. Der gebildete Niederschlag wurde abfiltriert.

Das Filtrat wurde durch Destillation von Wasser und Aceton befreit und der Destillationsrückstand analysiert:
Rhodium-Gehalt 2,5 ppm
Stickstoffgehalt 200 ppm

Der Niederschlag aus der Fällung wurde wieder mit der gleichen Menge an Dimerbuten versetzt und die Hydroformylierung unter den Bedingungen des Beispiels 5 wiederholt. Der Umsatz von Dimerbuten betrug 96,3 %, die Ausbeute an Nonanalen 84,4 % und die Ausbeute an Nonanolen 9,4 %.

### Beispiel 15

Kontinuierliche Hydroformylierung von Dimerbuten, Destillation, Ausfällung des Katalysators und des überschüssigen Liganden und Rückführung in die Hydroformylierung.

Dimerbuten wurde mit dem Katalysatorsystem Rhodium/Polyethylenimin (50 % Amidierung mit Stearinsäure gemäß Beispiel 3) kontinuierlich hydroformyliert. Der Reaktionsaustrag wurde in einem Wischblattverdampfer aufgetrennt, das Destillat und der Destillationssumpf wurden analysiert und der Destillationssumpf mit frischem Dimerbuten in die Hydroformylierung zurückgeführt.

Zulauf Olefin/Rücklauf Destillationssumpf (Massenverhältnis): 3/1; Temperatur der Hydroformylierung 150°C; Druck 280 bar CO/H₂ 1:1; Temperatur des Wischblattverdampfers 130°C; Druck im Wischblattverdampfer 10 mbar.

Rhodium-Gehalt des Destillationssumpfes 185 ppm Rh
Stickstoffgehalt des Destillationssumpfes 0,36 %
Gehalt an Hochsiedern im Destillationssumpf 90 %

10 % der Gesamtmenge des Destillationssumpfes wurden entnommen und mit der 9-fachen Masse Aceton und mit der 0,2-fachen Masse Wasser versetzt. Die Mischung wurde 30 min gerührt und 10 h bei 7°C gehalten. Der erhaltene Niederschlag wurde abfiltriert und das Filtrat wurde durch Destillation von Wasser und Aceton befreit. die Analyse des Destillationsrückstands ergab einen Rhodium-Gehalt von 3,1 ppm und einen Stickstoffgehalt von 280 ppm.

Der Filterrückstand wurde in einem weiteren Teil des Destillationssumpfes der kontinuierlichen Hydroformylierung aufgelöst und wieder in die Reaktion eingebracht. Diese Ausfällung wurde alle 24 h wiederholt. Nach 17 Tagen kontinuierlicher Hydroformylierung ohne zusätzlicher Zugabe von Rhodium und Ligand ergaben vor Beginn der Behandlung des Destillationssumpfes einen Umsatz von Dimerbuten von 91,1 %, eine Selektivität zu Aldehyden von 88,7 % und eine Selektivität zu Alkoholen von 8,7 %.

Nach 17 Tagen kontinuierlicher Versuchsführung wurde ein Umsatz von Dimerbuten von 89,5 %, eine Selektivität zu Aldehyden von 91,3 % und eine Selektivität zu Alkoholen von 6,7 % festgestellt.

### Beispiel 16

### Zweistufige Fällung

Mit dem gemäß Beispiel 14 erhaltenen Destillationsrückstand wurde eine zweistufige Fällung durchgeführt, um Katalysator und überschüssigen Liganden möglichst vollständig aus dem Destillationssumpf abzutrennen:

Das Filtrat der Fällung, hergestellt wie in Beispiel 14, wurde nicht eingeengt, sondern es wurden ihm weitere 2 Massen-% Wasser zugesetzt. Die Lösung wurde auf -30°C gekühlt und gerührt. Nach einer Stunde wurde eine kleine Menge eines Niederschlages abfiltriert. Das Filtrat wurde durch Destillation von Wasser und Aceton befreit und der Destillationsrückstand analysiert. Der Rhodium-Gehalt betrug nun 0,7 ppm und der Stickstoffgehalt 60 ppm. Ähnliche Ergebnisse werden erhalten, wenn die Destillationsrückstände aus der Hydroformylierung anderer Olefine gemäß den übrigen Hydroformylierungsbeispielen durch Fällung behandelt und rückgeführt werden.

### Beispiel 17

Kontinuierliche Hydroformylierung von Dimerbuten mit kontinuierlicher Ultrafiltration des Destillationssumpfes und Rückführung des Retentats enthaltend den Katalysator und überschüssigen Liganden in die Hydroformylierung.

Kontinuierliche Hydroformylierung von Dimerbuten mit einem modifizierten Polyethylenimin gemäß Beispiel 3.

In dem Reaktor R gemäß Fig. 1 wurde Dimerbuten mit dem Katalysatorsystem Rhodium/amidiertes Polyethylenimin kontinuierlich hydroformyliert. Der Reaktionsaustrag 2 wurde in einem Wischblattverdampfer K aufgetrennt. Über Kopf wurde der Produktstrom 3 abgezogen und der Destillationssumpf 4 kontinuierlich in der Membranfiltrationsanlage M aufgearbeitet. Das Retentat aus der Ultrafiltration 5 wurde in die Reaktion zurückgeführt. Die Verluste an Katalysator und Liganden, die mit dem Hochsieder in sehr geringer Menge permeieren, wurden durch Dosierung von entsprechend geringen Mengen Katalysator und Ligand in den Reaktor kompensiert.
Temperatur der Hydroformylierung 150°C
Druck 280 bar CO/H₂ 1:1
Temperatur des Wischblattverdampfers 130°C
Druck im Wischblattverdampfer 10 mbar

Der Destillationssumpf 4 enthielt 200 ppm Rh und 5 Gew.-% amidiertes Polyethylenimin. Diese Lösung wurde in einem Membranmodul, das mit einer Membran (ZrO₂, 15 000 Dalton) der Firma Tech-Sep ausgerüstet ist, einer Ultrafiltration bei 100°C, bei einem Transmembrandruck von 10 bar und einer Überströmgeschwindigkeit 2 m/s unterworfen.

Die Ergebnisse der Membranfiltration sind in der folgenden Tabelle zusammengestellt.

| | Ströme gemäß Fig. 1 | m (kg/h) | C_{Rhodium} (ppm) |
|---|---|---|---|
| 4 | (Sumpf) | 9 | 200 |
| 5 | (Retentat) | 6 | 294 |
| 6 | (Filtrat) | 3 | 12 |

Unter den Versuchsbedingungen betrug der spezifische Permeatfluß 20 kg/m²h. Die Rückhaltung von Rhodium betrug 95,9 %.

Diese Fahrweise wurde mehrmals wiederholt und führt im Durch-schnitt zu einem Dimerbutenumsatz von 89,5 %, mit einer Aldehyd-Selektivität von 89,3 % und einer Alkohol-Selektivität von 8 %.

Ähnliche Ergebnisse wurden erhalten, wenn Destillationsrückstände aus der Hydroformylierung anderer Olefine durch Ultrafiltration behandelt und rückgeführt wurden.

### Beispiel 18

Diskontinuierliche Hydroformylierung mit anschließender Ultrafiltration des Destillationssumpfes und Rückführung des den Katalysator und überschüssigen Liganden enthaltenden Retentats in die Hydroformylierung.

Diskontinuierliche Hydroformylierung von Dimerbuten mit einem amidierten Polyethylenimin gemäß Beispiel 3.

Beispiel 5 wurde mit der 10-fachen Einsatzmenge wiederholt und nach beendeter Umsetzung der Reaktionsaustrag bei 150°C und 10 mbar destilliert. Bei einer Rückstandsmenge von 200 g wurde die Destillation abgebrochen. Die Rhodiumkonzentration des Rück-standes betrug 125 ppm und die Konzentration an amidiertem Polyethylenimin 6,3 Gew. -%.

Der Destillationsrückstand wurde dann in einer einstufigen Membranfiltrationsanlage, die mit der Membran MPF-U20-S der Firma Membran Products Kiryat Weizmar ausgerüstet ist, aufgearbeitet.

Die Filtration lief bei 40°C, 9 bar und die Überströmgeschwindigkeit betrug 2 m/s. Die Ergebnisse der Membranfiltration sind in der folgenden Tabelle zusammengestellt.

| | Fig. 2 | m (g) | C_{Rhodium} (ppm) |
|---|---|---|---|
| B1 | (t=0) (Sumpf) | 200 | 125 |
| B1 | (t=t_{Ende}) (Retentat) | 100 | 242 |
| B2 | (Filtrat) | 100 | 8 |

Bei den angewandten Bedingungen betrug der spezifische Permeatfluß 1 kg/m²h. Die Rückhaltung von Rhodium betrug 96,7 %.

Das Retentat (100 g) wurde mit der gleichen Menge an Dimerbuten versetzt und die Hydroformylierung unter den Bedingungen des Beispiels 5 wiederholt. Der Umsatz von Dimerbuten betrug 97,8 %, die Ausbeute an Nonanalen 84,5 % und die Ausbeute an Nonanolen 9,3 %.

## Patentansprüche

1. Verfahren zur Herstellung von Aldehyden oder Aldehyden und Alkoholen durch Hydroformylierung von Olefinen in Gegenwart eines im Reaktionsgemisch homogen gelösten Komplexkatalysators, enthaltend ein Metall der Gruppe VIIIa des Periodensystems der Elemente und als Ligand eine phosphorfreie, mehrzähnige, zur Komplexbildung befähigte Stickstoffverbindung, bei Temperaturen von 50 bis 200°C und Drücken von 20 bis 1000 bar und Rückführung des Katalysatorkomplexes in die Hydroformylierungsreaktion, **dadurch gekennzeichnet, daß** man
a) als Liganden derivatisierte und zur Komplexbildung befähigte Polyamine mit einem mittleren Molekulargewicht größer 1000 Dalton und mit mindestens 10 Stickstoffatomen verwendet,die sich zu nicht mehr als 1 g/l in Wasser bei Raumtemperatur lösen und aus dem Reaktionsgemisch nicht mit Wasser extrahiert werden können,
b) aus dem Reaktionsgemisch nach Beendigung der Hydroformylierungsreaktion und destillativer Abtrennung oder partieller destillativer Abtrennung der Aldehyde und Alkohole den im Destillationssumpf verbleibenden Katalysatorkomplex und überschüssigen Liganden vollständig oder zum Teil in die Hydroformylierung zurückführt und
c) kontinuierlich oder zumindest absatzweise zumindest einen Teil der Hochsieder aus dem Sumpf der Destillation des Reaktionsgemisches ausschleust,
und hierbei
- den Katalysator und überschüssigen Liganden mit einem Lösungsmittel, in dem der Katalysator und der überschüssige Ligand unlöslich oder nahezu unlöslich ist, ausfällt und nur das Fällungsprodukt in die Hydroformylierung zurückführt, und/oder
- den Katalysator und überschüssigen Liganden durch Ultrafiltration des Destillationssumpfes gewinnt und den an Hochsiedern abgereicherten und an Katalysator und Liganden angereicherten Retentatstrom in die Hydroformylierung zurückführt, und/oder
- die im Destillationssumpf enthaltenen Hochsieder mittels Wasserdampfdestillation vom Katalysator und überschüssigem Liganden abtrennt und nur den nach der Wasserdampfdestillation erhaltenen, Katalysator und überschüssigen Liganden enthaltenden Destillationssumpf in die Hydroformylierungsreaktion zurückführt.

2. Verfahren gemäß Anspruch 1, **dadurch gekennzeichnet, daß** man als Polyamine Polyethylenimine bestehend im wesentlichen aus Einheiten der Formel I verwendet, 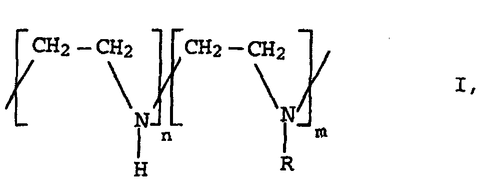 in der die Summe aus m + n mindestens 10 und das Verhältnis aus m/m+n 0,01 bis 1 beträgt und R gleiche oder verschiedene Alkyl-, Cycloalkyl-, Aryl-, Aralkyl-, Alkanoylgruppen mit bis zu 30 C-Atomen oder Hydroxyalkyl(poly)oxyalkylengruppen mit bis zu 500 Alkylenoxy-Einheiten bedeuten.

3. Verfahren gemäß Anspruch 1, **dadurch gekennzeichnet, daß** man als Liganden ein derivatisiertes Polyamin verwendet, das ein mittleres Molekulargewicht von mehr als 1000 Dalton aufweist.

4. Verfahren gemäß Anspruch 1, **dadurch gekennzeichnet, daß** man als Liganden ein derivatisiertes Polyamin verwendet, das einen Dampfdruck von weniger als 10⁻⁵ mbar bei 25°C hat.

5. Verfahren gemäß Anspruch 1, **dadurch gekennzeichnet, daß** man als Liganden ein derivatisiertes Polyamin verwendet, das als freier Ligand und in Form seines Komplexes mit dem Metall der Gruppe VIIIa im Reaktionsgemisch der Hydroformylierung vollständig löslich ist, aber in einem mit dem Reaktionsgemisch der Hydroformylierung mischbaren, polaren Lösungsmittel oder Gemischen aus mehreren solcher Lösungsmittel nicht oder nur wenig löslich ist, den Liganden und den Komplex des Liganden mit dem Metall der Gruppe VIIIa aus dem Sumpf oder einem Teil des Sumpfes der Destillation des Reaktionsgemisches aus der Hydroformylierung durch Zugabe dieses Lösungsmittels ausfällt und den so ausgefällten Liganden und Komplex des Liganden mit dem Metall der Gruppe VIIIa in die Hydroformylierungsreaktion zurückführt.

6. Verfahren gemäß Anspruch 5, **dadurch gekennzeichnet, daß** man ein polares Lösungsmittel ausgewählt aus der Gruppe bestehend aus Aceton, Tetrahydrofuran, Dimethylformamid, Dimethylacetamid, N-Alkylpyrrolidonen, Glykolen, Methanol, Ethanol, n-Propanol, Isopropanol und Mischungen dieser Lösungsmittel oder Mischungen dieser Lösungsmittel mit Wasser verwendet.

7. Verfahren gemäß Anspruch 1, **dadurch gekennzeichnet, daß** man nach Abdestillieren oder partiellem Abdestillieren der Aldehyde und Alkohole den Komplexkatalysator und überschüssige Liganden durch Ultrafiltration isoliert und in die Hydroformylierung zurückführt.

8. Verfahren gemäß Anspruch 1, **dadurch gekennzeichnet, daß** man während oder nach dem Abdestillieren oder partiellem Abdestillieren der Aldehyde und Alkohole den den Komplexkatalysator und überschüssigen Liganden enthaltenden Destillationssumpf einer Wasserdampfdestillation unterzieht und danach den Komplexkatalysator und überschüssigen Liganden enthaltenden Destillationssumpf vollständig oder zum Teil in die Hydroformylierung zurückführt.

9. Verfahren gemäß Anspruch 1, **dadurch gekennzeichnet, daß** man Olefine mit mehr als 3 C-Atomen hydroformyliert.

10. Verfahren gemäß Anspruch 1, **dadurch gekennzeichnet, daß** man Olefine mit 4 bis 24 C-Atomen hydroformyliert.

11. Verfahren gemäß Anspruch 1, **dadurch gekennzeichnet, daß** man die Hydroformylierung und die anschließende destillative Abtrennung der Aldehyde und Alkohole kontinuierlich durchführt, den größten Teil des den Katalysator, überschüssigen Liganden und die Hochsieder enthaltenden Sumpfes in die Hydroformylierungsreaktion zurückführt und einen geringen Teil des Sumpfes ausschleust, den Katalysator und überschüssigen Ligand daraus gemäß den Verfahren der Ansprüche 5, 7 oder 8 gewinnt oder in angereicherter Form gewinnt und ebenfalls in die Hydroformylierungsreaktion zurückführt.

## Claims

1. A process for preparing aldehydes or aldehydes and alcohols by hydroformylation of olefins in the presence of a catalyst complex which is homogeneously dissolved in the reaction mixture and comprises a metal of group VIIIa of the Periodic Table of the Elements and as ligand a phosphorus-free, polydentate nitrogen compound capable of complex formation, at from 50 to 200°C and pressures of from 20 to 1000 bar, and recycling of the catalyst complex to the hydroformylation reaction, wherein
a) the ligands used are modified polyamines which are capable of complex formation, have a mean molecular weight of greater than 1000 dalton, contain at least 10 nitrogen atoms, have a solubility in water of not more than 1 g/l at room temperature and cannot be extracted from the reaction mixture by means of water,
b) after the hydroformylation reaction is complete and the aldehydes and alcohols have been separated or partially separated from the reaction mixture by distillation, the catalyst complex and excess ligand remaining in the distillation bottoms are completely or partially recycled to the hydroformylation and
c) at least part of the high boilers are bled off, continuously or at least batchwise, from the bottoms from the distillation of the reaction mixture,
where
- the catalyst and excess ligand are precipitated by means of a solvent in which the catalyst and the excess ligand are insoluble or virtually insoluble and only the precipitation product is recycled to the hydroformylation, and/or
- the catalyst and excess ligand are recovered by ultrafiltration of the bottoms from the distillation and the retentate stream which is depleted in high boilers and enriched in catalyst and ligand is recycled to the hydroformylation, and/or
- the high boilers present in the bottoms from the distillation are separated from the catalyst and excess ligand by means of steam distillation and only the distillation bottoms comprising catalyst and excess ligand from the steam distillation are recycled to the hydroformylation reaction.

2. A process as claimed in claim 1, wherein the polyamines used are polyethylenimines consisting essentially of units of the formula I, 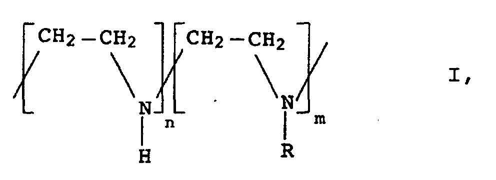 where the sum of m + n is at least 10 and the ratio of m/m+n is from 0.01 to 1 and R are identical or different alkyl, cycloalkyl, aryl, aralkyl or alkanoyl groups having up to 30 carbon atoms or hydroxyalkyl(poly)oxyalkylene groups having up to 500 oxyalkylene units.

3. A process as claimed in claim 1, wherein the ligand used is a modified polyamine which has a mean molecular weight of more than 1000 dalton.

4. A process as claimed in claim 1, wherein the ligand used is a modified polyamine which has a vapor pressure of less than 10⁻⁵ mbar at 25°C.

5. A process as claimed in claim 1, wherein the ligand used is a modified polyamine which is, both as free ligand and in the form of its complex with the metal of group VIIIa, completely soluble in the reaction mixture of the hydroformylation but is insoluble or only sparingly soluble in a polar solvent which is miscible with the reaction mixture of the hydroformylation or mixtures of a plurality of such solvents, the ligand and the complex of the ligand with the metal of group VIIIa is precipitated from the bottoms or part of the bottoms from the distillation of the reaction mixture from the hydroformylation by addition of this solvent and the ligand and complex of the ligand with the metal of group VIIIa which have been precipitated in this way are recycled to the hydroformylation reaction.

6. A process as claimed in claim 5, wherein use is made of a polar solvent selected from the group consisting of acetone, tetrahydrofuran, dimethylformamide, dimethyl- acetamide, N-alkylpyrrolidones, glycols, methanol, ethanol, n-propanol, isopropanol and mixtures thereof with or without water.

7. A process as claimed in claim 1, wherein, after distilling off or partially distilling off the aldehydes and alcohols, the catalyst complex and excess ligands are isolated by ultrafiltration and recycled to the hydroformylation.

8. A process as claimed in claim 1, wherein, while or after distilling off or partially distilling off the aldehydes and alcohols, the distillation bottoms comprising the catalyst complex and excess ligand are subjected to a steam distillation and the distillation bottoms comprising the catalyst complex and excess ligand are then completely or partially recycled to the hydroformylation.

9. A process as claimed in claim 1, wherein olefins having more than 3 carbon atoms are hydroformylated.

10. A process as claimed in claim 1, wherein olefins having from 4 to 24 carbon atoms are hydroformylated.

11. A process as claimed in claim 1, wherein the hydroformylation and the subsequent distillation to separate off the aldehydes and alcohols are carried out continuously, the major part of the bottoms comprising the catalyst, excess ligands and the high boilers is recycled to the hydroformylation reaction and a small part of the bottoms is bled off, the catalyst and excess ligand are recovered or recovered in enriched form therefrom by means of a process as claimed in claim 5, 7 or 8 and likewise recycled to the hydroformylation reaction.

## Revendications

1. Procédé de préparation d'aldéhydes ou d'aldéhydes et d'alcools par hydroformylation d'oléfines en présence d'un catalyseur complexe dissous de manière homogène dans le mélange réactionnel, contenant un métal du groupe VIIIa de la table Périodique des Eléments et comme coordinat une combinaison azotée multiple exempte de phosphore capable de former un complexe, à des températures de 50 à 200°C et sous des pressions de 20 à 1000 bars, et renvoi du complexe de catalyseur dans la réaction d'hydroformylation, **caractérisé en ce que**:
a) on utilise comme coordinat des dérivés de polyamines capables de former des complexes, d'un poids moléculaire moyen supérieur à 1000 daltons et comportant au moins 10 atomes d'azote qui ne se dissolvent pas dans l'eau à raison de plus de 1g/l à la température ambiante et ne peuvent pas être extraits avec de l'eau du mélange réactionnel,
b) on renvoie, du mélange réactionnel, une fois la réaction d'hydroformylation et la séparation par distillation ou la séparation par distillation partielle des aldéhydes et des alcools terminées, le complexe de catalyseur et le coordinat en excès restant dans le fond de distillation, entièrement ou en partie, dans la réaction d'hydroformylation, et
c) on évacue en continu ou tout au moins par étapes au moins une partie des substances de point d'ébullition élevé du fond de distillation du mélange réactionnel,
et ainsi
• on précipite le catalyseur et le coordinat en excès avec un solvant, dans lequel le catalyseur et le coordinat en excès sont insolubles ou presqu'insolubles, et on renvoie seulement le produit de précipitation dans la réaction d'hydroformylation, et/ou
• on récupère le catalyseur et le coordinat en excès par ultrafiltration du fond de distillation et on renvoie dans la réaction d'hydroformylation le courant retenu appauvri en substances de point d'ébullition élevé et enrichi en catalyseur et coordinat, et/ou
• on sépare les substances de point d'ébullition élevé contenues dans le fond de distillation par entraînement à la vapeur, du catalyseur et du coordinat en excès, et on renvoie seulement dans la réaction d'hydroformylation le fond de distillation contenant le catalyseur et le coordinat en excès obtenu après l'entraînement à la vapeur.

2. Procédé selon la revendication 1, **caractérisé en ce que** on utilise comme polyamines des polyéthylènamines constituées essentiellement d'unités répondant à la formule 1 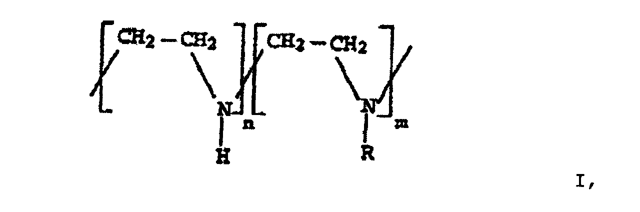 dans laquelle la somme de m + n est au moins 10 et le rapport m/m+n est 0,01 à 1 et R représente des groupes alkyle, cycloalkyle, aryle, aralkyle, alcanoyle, identiques ou différents, comportant jusqu'à 30 atomes de C ou des groupes hydroxyalkyl(poly)oxyalkylène comportant jusqu'à 500 unités alkylènoxy.

3. Procédé selon la revendication 1, **caractérisé en ce qu'**on utilise, comme coordinat, un dérivé de polyamine, qui présente un poids moléculaire moyen supérieur à 1000 daltons.

4. Procédé selon la revendication 1, **caractérisé en ce que** l'on utilise, comme coordinat un dérivé de polyamine qui présente une pression de vapeur inférieure à 10⁻⁵ mbar à 25°C.

5. Procédé selon la revendication 1, **caractérisé en ce que** l'on utilise comme coordinat un dérivé de polyamine, qui est entièrement soluble en tant que coordinat libre et sous forme de son complexe avec le métal du Groupe VIIIa dans le mélange réactionnel de l'hydroformylation, mais n'est pas soluble ou est seulement peu soluble dans un solvant polaire ou un mélange de plusieurs solvants de ce genre, miscible avec le mélange réactionnel de l'hydroformylation, on précipite le coordinat et le complexe du coordinat avec le métal du Groupe VIIIa, du fond de distillation ou d'une partie du fond de distillation du mélange réactionnel de l'hydroformylation par addition de ce solvant, et on renvoie le coordinat et le complexe du coordinat avec le métal du groupe VIIIa ainsi précipités dans la réaction d'hydroformylation.

6. Procédé selon la revendication 5, **caractérisé en ce que** l'on choisit un solvant polaire appartenant au groupe constitué d'acétone, tétrahydrofuranne, diméthylformamide, diméthylacétamide, N-alkylpyrrolidones, glycols, méthanol, éthanol, n-propanol, isopropanol et de mélanges de ces solvants ou de mélanges de ces solvants avec de l'eau.

7. Procédé selon la revendication 1, **caractérisé en ce que** on l'isole, après distillation ou distillation partielle des aldéhydes et des alcools, le catalyseur complexe et le coordinat en excès par ultra-filtration et qu'on les renvoie dans l'hydroformylation.

8. Procédé selon la revendication 1, **caractérisé en ce que** l'on soumet, pendant ou après la distillation ou la distillation partielle des aldéhydes et des alcools, le fond de distillation contenant le catalyseur complexe et le coordinat en excès à un entraînement par vapeur d'eau et qu'on renvoie ensuite le fond de distillation contenant le catalyseur complexe et le coordinat en excès, entièrement ou en partie, dans l'hydroformylation.

9. Procédé selon la revendication 1, **caractérisé en ce que** l'on hydroformyle des oléfines comportant plus de 3 atomes de C.

10. Procédé selon la revendication 1, **caractérisé en ce que** l'on hydroformyle des oléfines comportant 4 à 24 atomes de C.

11. Procédé selon la revendication 1, **caractérisé en ce que** l'on l'effectue en continu l'hydroformylation et la séparation par distillation qui lui fait suite des aldéhydes et des alcools, on renvoie la plus grande partie du fond contenant le catalyseur, le coordinat en excès et les substances de point d'ébullition élevé dans la réaction d'hydroformylation et on évacue une petite partie du fond, on récupère de celui-ci le catalyseur et le coordinat en excès selon le procédé des revendications 5, 7 ou 8 ou on les récupère sous forme enrichie et on les renvoie également dans la réaction d'hydroformylation.
